# EUROPEAN PATENT SPECIFICATION

(11) **EP 0 830 604 B1**
(45) Date of publication and mention of the grant of the patent: **28.05.2003**
(21) Application number: 96916378.1
(22) Date of filing: 07.06.1996
(51) Int. Cl.: G01N 33/574, A61K 38/16, C12N 15/34, C07K 14/01

(54) **USES OF APOPTIN**
VERWENDUNGEN VON APOPTIN
UTILISATIONS DE L'APOPTINE

(30) Priority: 07.06.1995 US 484939
(43) Date of publication of application: 25.03.1998
(73) Proprietor: Leadd B.V., 2333 AL Leiden (NL)
(72) Inventor: NOTEBORN, Matheus, Hubertus, Maria, NL-2352 EH Leiderdorp (NL)
(74) Representative: Smulders, Theodorus A.H.J., Ir.
(86) International application number: NL9600229
(87) International publication number: WO96041191

(56) References cited:
- WO-A-95/03414
- US-A- 5 981 502
- CANCER RESEARCH, vol. 55, February 1995, pages 486-489, XP000602162 ZHUANG S.-M. ET AL: "Apoptin, a protein derived from chicken anemia virus, induces p53-independent apoptosis in human osteosarcoma cells" cited in the application
- JOURNAL OF VIROLOGY, vol. 68, no. 1, 1994, pages 346-351, XP002013505 NOTEBORN M.H.M. ET AL: "A single chicken anemia virus protein induces apoptosis"
- JOURNAL OF VIROLOGY, vol. 65, no. 6, 1991, pages 3131-3139, XP002013506 NOTEBORN M.H.M. ET AL: "Characterization of cloned chicken anemia virus DNA that contains all elements for the infectious replication cycle"
- AVIAN PATHOLOGY, vol. 24, 1995, pages 11-31, XP000577477 NOTEBORN M.H.M. ET AL: "Chicken anemia virus infection: molecular basis of pathogenicity" cited in the application
- LEUKEMIA, vol. 9, no. 1, October 1995, pages s118-s120, XP000602147 ZHUANG S.-M. ET AL: "Apoptin, a protein encoded by chicken anemia virus, induces cell death in various human hematologic malignant cells in vitro" cited in the application
- PubMed Abs., PMID 2445861, (1987)
- PubMed Abs., PMID 9801139, (1998)
- PubMed Abs., PMID 2156209, (1990)
- PubMed Abs., PMID 9768359, (1998)
- PubMed Abs., PMID 8314906, (1993)
- Gene Therapy, vol.6, p.882-892, (1999)

## Description

### BRIEF DESCRIPTION OF THE INVENTION.

The present invention relates to methods and uses for apoptin or derivatives or functional fragments thereof, whereby apoptin stands for the viral protein 3 (VPS) of the chicken anaemia virus. Apoptin itself and its apoptosis inducing activity have been disclosed before (see below). However, in those earlier publications apoptin was no different from any other apoptosis inducing agent. We have now found that apoptin is indeed very different from other apoptosis inducing agents and may therefore be applied in different methods and for different uses. Whereas conventional apoptosis inducing agents induce apoptosis in any cell in which they are present we have now found that apoptin induces apoptosis only in transformed cells or tumor cells.

Thus the invention in one embodiment provides targeted anti-tumor therapies. The application of apoptin as anti-tumor therapy will cause little toxicity for apoptin induces cell death to a high extent in tumor cells and much reduced if at all in normal non-transformed, non-malignant cells.

The invention also discloses that apoptin cannot be inhibited by several anti-apoptosis inhibitors. In particular, apoptin acts distinct from the p53-apoptotic pathway, which is known to be a necessary element in mediating apoptosis, which is triggered by a variety of (chemo)-therapeutic agents.

The invention further discloses the finding that apoptin can induce apoptosis in various types of human tumor cells.

The invention further relates to differences in localization of apoptin in cells susceptible for apoptin-induced apoptosis, viz. human transformed and malignant cells, versus cells insensitive to apoptin-induced apoptosis, viz. human normal cells. The differential location is used as diagnostic assay to analyse whether a cell is normal or has become transformed and/or malignant.

### BACKGROUND OF THE INVENTION.

Jeurissen et al. (1992b) observed a number of phenomena in the thymi of chicken anemia virus (CAV)-inoculated specific-pathogen-free chickens that were absent in thymi of control chicken. Ten days after infection, the entire cortex contained cells whose chromatin had condensed in areas adjacent to the nuclear membrane. More or less spherical electron-dense bodies were seen sporadically in the cytoplasm of epithelial cells. At day 13 after infection, the cortex was severely depleted of thymocytes, whereas epithelial cells, many of them containing electron-dense bodies and other non-lymphoid cells were still present. DNA isolated from thymuses of chickens infected with various field isolates of CAV displayed the typical laddering of oligonucleosomal breakdown in an electropherogram. These observed morphological cellular and biochemical changes were also observed in CAV-infected cultured avian lymphoblastoid cells.

The above phenomena are characteristic of the physiological process of programmed cell death or apoptosis. Apoptosis is characterized by shrinkage of cells, segmentation of the nucleus, condensation and cleavage of DNA into domain-sized fragments, in most cells followed by internucleosomal degradation. Finally, the apoptotic cells fragment into membrane-enclosed bodies, which are rapidly phagocytosed by neighboring cells. Therefore, apoptosis causes much less destruction of tissue than necrosis, the non-physiological type of cell death (Wyllie et al., 1980, Arends and Wyllie, 1991).

Apoptosis is a cascade of events. In general, the apoptotic process can be devided in several stages.
**Stage 1. Triggering of apoptosis.**
   Many different external and internal agents can trigger the apoptotic process.
**Stage 2. Factors which can mediate the apoptotic trigger.**
   A main role at this stage is played by e.g. the tumor-suppressor protein p53.
**Stage 3. Enhancing of the apoptotic signal by factors as Bax.**
**Stage 4. Activation of the ICE serine-protease family members.**
   As soon as these specific proteases are activated, the point of no return has been passed. Known apoptosis inhibitors as growth factors (inhibits at stage 2), Bcl-2 (stage 3) or crmA (stage 4) are known to inhibit apoptosis at different stages during the decission stage of the apoptotic process
**Stage 5. Execution of the apoptotic signal. For instance, DNA becomes condensed and fragmented.(White, 1996).**

Early after infection of cultured chicken mononuclear cells, the CAV-encoded protein apoptin (also called VP3) co-localizes with cellular chromatin. Later after infection, apoptin forms aggregates, the cells become apoptotic, i.e. the cellular DNA is fragmented and, as a result, becomes condensed (Noteborn et al., 1994). It was shown by immunogold electron microscopy that apoptin is present in apoptotic structures. In vitro, expression of apoptin in chicken transformed lymphoblastoid T cell and myeloid cell induced apoptosis in these cells. These data indicate that apoptin can trigger the apoptotic pathway in CAV-infected cells (Noteborn et al. 1994, Noteborn and Koch, 1995).

Apoptin is a small protein, only 121 amino acids long, which is rather basic and proline-rich (Noteborn et al., 1991). Apoptin is located strictly within the cellular chromatin structures. Truncation of the C-terminal basic stretch of apoptin results in a reduced nuclear location and a significantly reduced apoptotic activity (Noteborn et al., 1994). The small size and rather basic character of apoptin may allow it to interact with histone and/or non-histone proteins within the chromatin structure.

Apoptosis is an active and programmed physiological process for eliminating superfluous, altered or malignant cells (Earnshaw, 1995). The apoptotic process can be initiated by a variety of regulatory stimuli (Wyllie, 1995 and White, 1996). Changes in the cell survival rate play an important role in human pathogenesis, e.g. in cancer development, which is caused by enhanced cell proliferation but also by decreased cell death (Kerr et al., 1994). A variety of chemotherapeutic compounds and radiation have been demonstrated to induce apoptosis in tumor cells, in many instances via wild-type p53 (Thompson, 1995, Bellamy et al., 1995, Steller, 1995, Kaufman, 1989, McDonell et al., 1995, Lowe et al. and Fisher, 1994).

Many tumors, however, acquire a mutation in p53 during their development, often correlating with poor response to cancer therapy (Hooper, 1994). For several (leukemic) tumors, a high expression level of the proto-oncogene Bcl-2 is associated with a strong resistance to various apoptosis-inducing chemotherapeutic agents (Hockenberry, 1994, Kerr et al., 1994, Sachs and Lotem, 1993).

Apoptin can induce apoptosis human malignant cell lines (Noteborn and Koch, 1994). We have established that apoptin-induced apoptosis occurs in the absence of functional p53 (Zhuang et al., 1995a), and cannot be blocked by Bcl-2 and BCR-ABL (Zhuang et al., 1995). Therefore, apoptin is useful for the destruction of tumor cells, which have become resistant to (chemo)therapeutic induction of apoptosis, due to the lack of functional p53 and (over)-expression of Bcl-2 and BCR-ABL.

### DETAILED DESCRIPTION OF THE INVENTION.

The invention discloses the apoptottc activity of apoptin in human malignant and transformed cells versus normal cells. Apoptin fails to induce apoptosis in primary T cells, endothelial and smooth muscle cells. When normal cells are transformed they become susceptible by apoptin. In normal cells apoptin was found predominantly in the cytoplasm, while in tumor cell it was located in the nucleus.

Apoptin can be used for depletion of specifically transformed/malignant cells. Therefore, apoptin is a very potent anti-tumor agent. Expression of apoptin can be used for the induction of apoptosis in specifically human tumor cells. Apoptin does not or at least not significantly induce apoptosis in normal cells, indicating that the toxicity of apoptin-treatment will be low.

The invention discloses that apoptin is able to induce apoptosis in transformed, non-immortalized human cells, which implies that the apoptotic activity of apoptin becomes already available during early transformation events in cells. Therefore, apoptin can be included in other (e.g. gene- or chemo-) therapies to prevent treated cells for undergoing transformation and even malignancy.

Apoptin can be (transiently) expressed in tumors by means of DNA transfection. Expression of apoptin in (tumor) cells may also take place by infecting cells with (retro)viral vectors that contain a coding sequence for apoptin.

Administration to cells of non-viral components (e.g. liposomes or transferrin-derived vectors) coding sequence for apoptin is a further possibility for the expression/presence of apoptin and induction of apoptosis in tumor cells.

Furthermore, we have provided evidence that. apoptin is distinct from the p53-induced apoptotic pathway. Besides the fact that apoptin does not need functional p53, apoptin cannot be blocked by inhibitors of the p53-apoptotic pathway, as the Bcl-2(-like) proteins, and the cow-pox protein crmA, which blocks the ICE-like protease activities. These inhibitors interfere within different stages of the p53-induced apoptotic cascade Apoptin, might induce apoptosis completely independent from the apoptotic pathway blocked by these apoptotic inhibitors, or act downstream from them.

These data imply that apoptin is a very potent inducer of apoptosis in tumor cells, which can overcome (all analysed) blocks of anti-apoptotic activity in transformed and malignant cells. Therefore, apoptin is a potent anti-tumor agent for a broad variety of tumor(s) (cells).

The differential localization of apoptin in normal versus transformed/malignant cells can be used as diagnostic test to distinguish whether a cell has become transformed/malignant.

Besides, the induction of apoptosis in human tumor cells, we have also provided evidence that apoptin can induce apoptosis in other mammalian tumor cells. The invention can be used for treatment of cancer in various human sytems.

Thus the invention provides the use of a vehicle delivering a gene encoding a tumoricidal substance mainly but not exclusively with a human tumor cell for preparing a medicament for preventing malignancies by delivering the gene to transformed non-immortalized cells, characterized in that the tumoricidal substance is apoptin or a functional equivalent thereof. In the prior art many vehicles for delivering cytotoxic agents or precursors therefor have been disclosed. A major problem in delivering cytotoxic agents is that they are harmful to all cells and not just the tumor cells (which will be interchangeably called transformed or malignant or tumor cells herein). Therefore many different ways of targetting the cytotoxic substance to the tumor cells have been investigated. Although many targetting moieties are nowadays known, all of them suffer from the drawback of not being completely specific for the tumor target. Therefore the use of this cytotxic substances has sofar not met with great success. At the time of its discovery apoptin was thought to suffer from the same drawback in that it would need a highly specific targetting moiety. We have now found that this is not necessary for apoptin. Apoptin only has a significant effect in tumor cells and not in normal cells. Thus, even if the targetting moiety or any other means of delivery of apoptin (or a functional equivalent) is not very specific, this will result in hardly any toxicity to normal tissue. A functional equivalent of apoptin is any fragment or derivative having the same kind of activity, possibly in different amounts.

A targetting moity is well defined in the art as a molecule with a specific binding activity for a target molecule. It should preferably be capable of internalization. The target molecule may be an antigen or an epitope, in which case the targetting moity is an antibody or a fragment or a derivative thereof. The target molecule may be a receptor in which case the targetting moiety is a ligand for said receptor. These are just examples of suitable combinations. The bond between the targetting moiety and the apoptin has only one requirement in that it should allow the functions of both partners to operate. Thus, it may be a chemical (labile) bond, it may be a fusion protein. The conjugate may even be a liposome covered with targetting moieties and filled with apoptin (or a gene encoding it) etc. The invention also comprises the use of a vehicle for preparing a medicament for use in delivering a gene encoding apoptin to a human tumor cell, using gene therapy wherein the vehicle comprises a targeting moiety having affinity for a molecule associated mainly, but not exclusively with the surface of said tumor cell. Gene therapy has many well known methods to deliver genes to cells using viruses such as adenovirus or retrovirus. The person skilled in the art will know how to select the right vehicle. Because apoptin only functions to induce apoptosis in transformed cells, it or its gene can be used as a safety measure in other gene therapy regimens than tumor therapy, such as those rectifying deficiencies from inheritable diseases. It is then provided in a gene delivery vehicle together with the gene of interest and if the cell in which the gene of interest is inserted becomes malignant then it will be susceptible to the action of apoptin.

Apoptin cannot be inhibited by the mechanims inhibiting the other apoptosis inducing substances. If apoptin is to be used to eliminate cells that are not transformed then this can be achieved by providing apoptin or a functional equivalent thereof provided with a nuclear localization signal. Since apoptin needs to be in the nucleus this will result in apoptosis. Without said signal the apoptin would remain outside the nucleus and have no significant effect (as disclosed herein).

Thus said apoptin with a nucleus localization signal could be used in a method for elimination of cells of a population of target cells. Nuclear localization signals (NLS) are well known in the art. One can provide a fusion gene encoding an NLS and an apoptin.

Because apoptin has a different localization in normal cells as compared to in transformed cells, it is possible to use apoptin as a diagnostic tool distinguishing between those two kinds of cells. Thus the invention also provides a method for distinguishing between transformed and/or malignant and/or tumor cells and normal cells comprising the steps of providing said cells with the viral protein 3 (VP3; apoptin) and detecting the presence or absence of apoptotic activity of apoptin eg. by detecting the localization of said viral protein in said cells. The localized apoptin can be detected with e.g. antibodies.

The invention will be explained in more detail on the bassis of the following experimental part. This is only for the purpose of illustration and should not be interpreted a a limitation of the scope of protection.

### EXPERIMENTAL

### Isolation of human primary cells.

Human primary T cells were isolated from 6 normal blood donors by Ficol centrifugation and grown in RPMI-medium containing 6% human serum and 0.8 ug per ml phytohemagglutinin. After 3 days the medium was refreshed and 300 units per ml Interleukin-2 was added. Human primary smooth-muscle cells (SMC) and vascular endothelial cells (HUVEC) were isolated from umbilical cords. SMC were grown in CM199 medium supplemented with ECGF and heparine and HUVEC in DMEM medium containing 10% fetal calf serum.

### DNA plasmids.

All CAV DNA sequences are originally derived from the plasmid DNA pIc-20H/CAV-EcoRI (Noteborn and De Boer, 1990). All cloning steps with plasmid DNAs were in principle carried out according the methods by Maniatis et al. (1982).

The expression plasmid pCMV-fs, formerly called pCMV-VP3, contains CAV DNA sequences encoding apoptin exclusively (nt 427-868), plasmid pCMV-tr encodes a truncated apoptin which lacks the C-terminal 11 amino acids (Zhuang et al., 1995a), and pCMV-des encodes desmin, a structural protein found in muscle cells that does not induce apoptosis (Menke et al., unpublished data). The expression plasmids pCMV-BAG-1 (Noteborn, M.H.M., unpublished data), pCMV-BcL-2 (Zhuang et al., 1995b) and pCMV-crmA (Noteborn, M.H.M., unpublished data) expresses the anti-apoptosis proteins BAG-1, Bcl-2 and the cowpox crmA protein inhibiting ICE-like proteases, respectively. The expression plasmids pCMV-E1B21K expresses the adenovirus E1B 21kDa protein and pCMV-p53, the tumor supressor protein p53 (Zhuang et al., 1995).

All transiently expressed genes are under the regulation of the cytomegalovirus promoter.

### Human normal, transformed and malignant keratinocytes and fibroblasts.

Human keratinocytes were isolated from foreskin and grown in the presence of a layer of mouse 3T3 fibroblasts lethally irradiated with 137Cs. Primary cultures of human epidermal keratinocytes (FSK-1) were initiated in complete medium as described (Rheinwald and Green, 1975) with minor modifications.

Human normal foreskin diploid VH10 fibroblasts (Klein, 1990) were grown in DMEM supplemented with 10% fetal-calf serum.

SV40-transformed tumorigenic NW18 fibroblasts (Weissman and Stanbridge, 1983) were grown in MEM medium supplemented with 8% fetal calf serum.

Tumorigenic keratinocytes, SCC-15 (Rheinwald and Beckett, 1981), derived from squamous-cell carcinoma, were cultured in DMEM/F12 (3:1) medium containing 5% fetal-calf serum, 0.4 ug per ml hydrocortisone and 1 uM isoproterenol.

The SV40-transformed fibroblasts, pre-crisis (Pre) and post-crisis (Post), as described by B. Klein et al. (1990) were grown in MEM medium with 8% fetal-calf serum.
The spontaneously transformed keratinocyte strain HaCaT (Boukamp et al., (1988) was a gift from Prof. Dr. Fusenig, DKFZ, Heidelberg, Germany. HaCaT cells were grown in DMEM medium supplemented with 10% fetal-calf serum. The SV40-transformed keratinocyte strain SVK14 (Taylor-Papadimitriou et al., 1982) cells were cultured in the same medium as SCC-15 cells.

### DNA transfections.

Plasmid DNA was purified by centrifugation in a CsCl gradient and column chromatography in Sephacryl S500 (Pharmacia).

Phytohemagglutinin-stimulated primary human T cells were transfected in the presence of DEAE-dextran, as described (Noteborn et al. 1994). Mouse Crip cells and human Hep3B, VH10, Pre- and Post-, and NW18 cells, HUVECs and SMCs were transfected with plasmid DNA by calcium-phosphate precipitation as described (Graham and Van der Eb, 1973). FSK-1, HaCaT, SVK14 and SSC-15 cells were transfected with DOTAP [D.Fischer, unpublished results]

### Immunofluorescence

T cells were grown in suspension and fixed on glass microscope slides. All other cells were grown on coated glass microscope slides. The cells were fixed with 80% acetone for 10 min at room temperature, and used for indirect immunofluorescence as described (Noteborn et al., 1994). To demonstrate the presence and/or cellular location of (truncated) apoptin in transfected cells mouse monoclonal antibodies (MAb) CVI-CAV-85.1-(85.1) (Noteborn et al., 1991) and CVI-CAV-111.3 (111.3; Koch, unpublished data) were used and for human desmin the mouse MAb 33 (Monosan, Uden, The Netherlands) was used. Fluorescein-isothiocyanate-labeled goat anti-mouse antibodies (Jackson Immunoresearch Laboratories Inc., West Grove PA) were used as second antibodies. Nuclear DNA was stained with 2,4-diamidino-2-phenylindole (DAPI) or propidium iodide (PI).

### Stable DNA tranfection of VH10 cells.

Human normal diploid VH10 fibroblasts were stably transfected with pCMV-fs, expressing full-sized apoptin or pCMV-neo-Bam, the empty plasmid without the CAV sequences encoding apoptin. Stable clones were selected with G418 medium, grown on glass microscope slides and fixed with 80% acetone. Expression of apoptin was analysed by indirect immunofluorescence using MAb 85.1.

### RESULTS AND DISCUSSION

### Expression of apoptin in mouse tumor cells in vitro.

We have examined whether apoptin can induce apoptosis also in mammalian tumor cells of non-human origin. Therefore, cells of the mouse tumor cell line Crip (Danos, 1988) werxe transfected with pCMV-fs DNA encoding apoptin. Three days after transfection the cells were fixed. By meansof immunofluorescence and PI staining the cells were examined for expression of apoptin, and whether they became apoptotic. Already, at three days after transfection 54% of the mouse tumor cells, which contain apoptin, have become apoptotic.

These results indicate that apoptin can induce apoptosis in different mammalian tumor cell lines.

### In Hep 3B cells apoptin-induced apoptotic pathway is distinct from the p53-induced apoptotic pathway.

Recently, we have shown that apoptin induces apoptosis in osteosarcoma cells, without the presence of wild-type p53 (Zhuang, et al., 1995, Noteborn and Koch, 1994). Chiou et al. (1994) and Debbas and White (1993) have provided evidence that Bcl-2 and adenovirus 5 (Ad 5) E1B 21K protein can block p53-induced/mediated apoptosis by acting downstream from p53.

We examined whether Ad 5 E1B 21K protein and Bcl-2 can inhibit the p53-independent pathway of apoptosis induced by apoptin in comparison to p53-dependent apoptosis. To that end, we studied the effect of co-expression of these proteins with apoptin or p53 in the human hepatoma Hep3B cell line.

Hep3B cells were co-transfected with pCMV-fs, encoding apoptin and pCMV-neo-Bam DNA (negative control), PCMV-E1B21 DNA, encoding the Ad5 E1B 21K protein or pCMV-Bcl2 (Zhuang et al., 1995b), encoding human Bcl-2. The number of apoptin-expressing cells was screened by indirect immunofluorescence and by DAPI-staining, which is weak and/or irregular when the cells have become apoptotic.

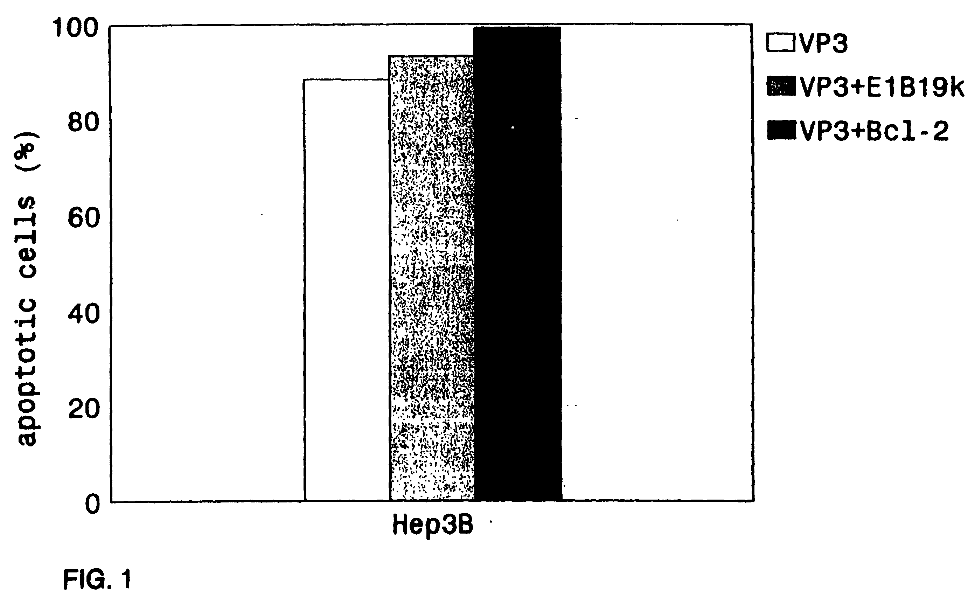

Surprisingly, at several timepoints after transfection the percentage of apoptin-expressing Hep3B cells, which became apoptotic was similar to the Hep3B cells containing both apoptin and E1B-21K or both apoptin and Bcl-2. For the sake of brevity, only the data obtained six days after transfection are given in Figure 1.

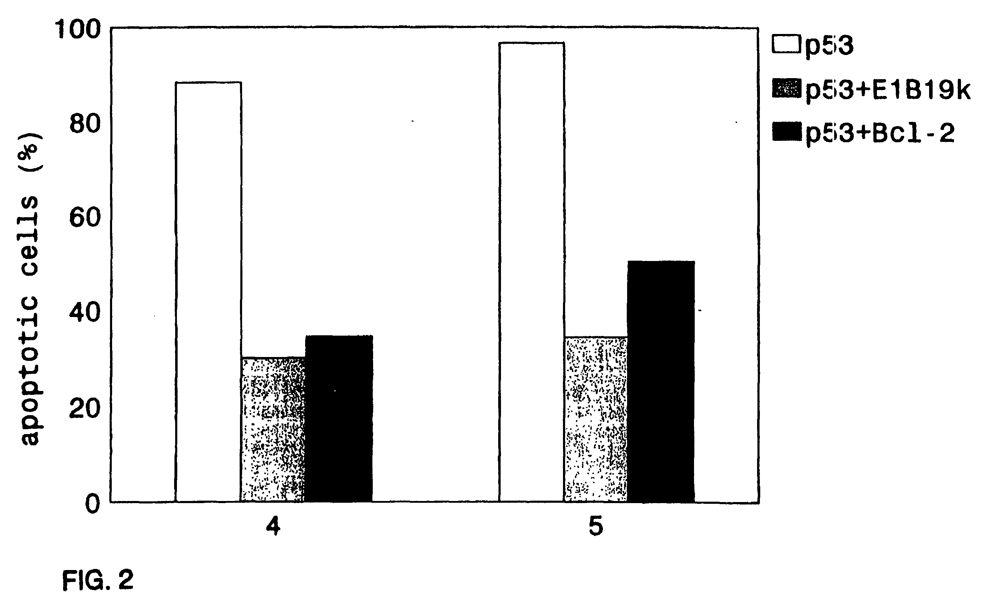

To illustrate that E1B-21K or Bcl-2 indeed have an anti-apoptotic effect in Hep3B cells, we examined whether these two proteins could inhibit apoptosis caused by over-expression of p53 in Hep3B cells. Compared to co-transfection of Hep3B cells with pCMV-p53 DNA encoding wild-type p53 (Baker et al., 1990) and pCMV-neo-Bam, co-transfection with plasmids pCMV-p53 and pCMV-E1B21, or pCMV-Bcl2 resulted in a significant reduction of apoptosis induced by p53, as analysed by immunofluorescence and PI-staining (Figure 2).

Thus, absence of effect of E1B 21K and Bcl-2 proteins on apoptin-induced apoptosis can not be explained by non-functionality of the expressed proteins in Hep3B cells for they can inhibit p53-induced apoptotic pathway. The fact that E1B 21K and Bcl2 still could negatively influence the p53-regulated apoptotic pathway in Hep3B cells, although the apoptin-induced apoptosis could not be inhibited indicates that the p53-dependent and apoptin-inducable p53-independent apoptotic pathway are distinct routes at least within Hep3B cells.

The fact that in a large number of tumors apoptosis cannot be induced in a variety of chemotherapeutic agents seems to be related to disrupted functions of p53 (Lowe et al., 1993). Therefore, induction of a p53-independent apoptotic pathway is a useful approach as alternative for tumor therapy.

### Characterization of the apoptin-induced apoptotic pathway in Saos-2 cells.

The apoptin-induced apoptotic pathway seems to be distinct from that induced by p53 (see above). Bcl-2, known to be involved in tumorformation via inhibition of the apoptotic pathway, could not block apoptin-induced apoptosis, but could block the p53-pathway. Recently, it has been reported by others that Bcl-2 and Bag-1 (Takayama et al., 1995) together might be required for the inhibition of so-called Bcl-2-independent apoptosis, as seems to be the case for apoptin-induced apoptosis.

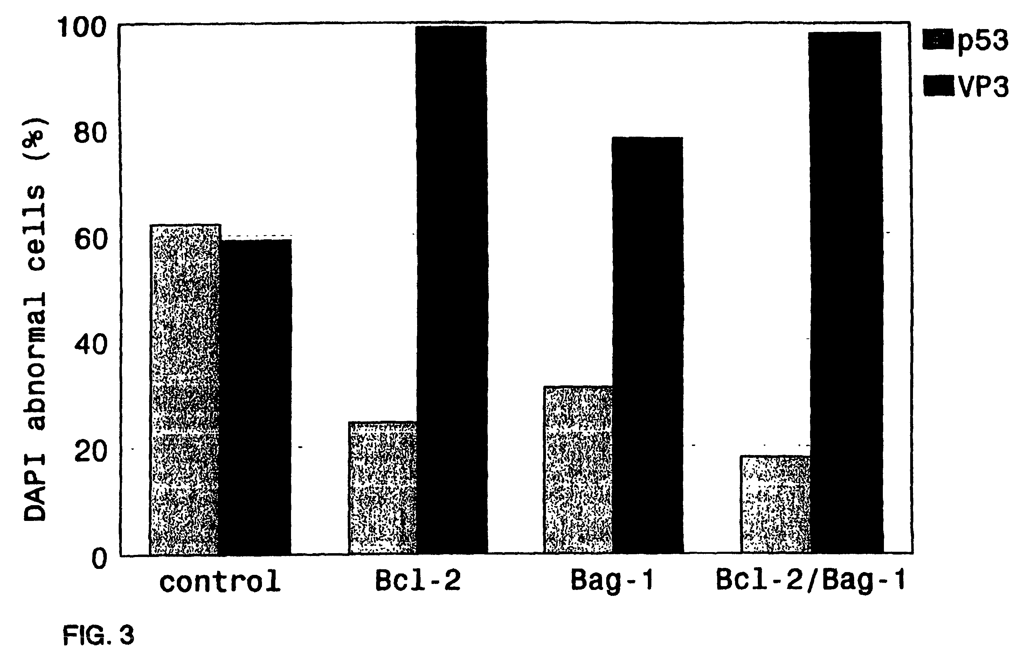

To examine whether Bag-1 could negatively influence the apoptin-induced apoptosis on its one or together with Bcl-2, co-transfections of Saos-2 cells pCMV-fs and pCMV-Bcl-2, or pCMV-Bag-1, or pCMV-Bcl-2 and pCMV-Bag-1 were carried out. In parallel, a controls, similar co-transfection experiments were carried out with p53. Bag-1 or a combination of Bag-1 and Bcl-2 did not inhibit apoptin-induced apoptosis, whereas p53-induced apoptosis was significantly inhibited by Bag-1 or Bag-1 and Bcl-2 (Figure 3). We conclude that the apoptin-induced apoptotic pathway is independent from Bcl-2-like proteins or acts downstream from them.

By double-immunofluorescence and video-intensified fluorescence microscopy was analysed whether apoptin could regulate bax-expression. Bax is a cellular protein, which can induce apoptosis and associates to Bcl-2 proteins and other, yet unknown, cellular proteins. p53 upregulates the expression of Bax, which results in induction of apoptosis (Oltvai et al. 1993). We have found evidence that apoptin did not upregulate the Bax protein level, whereas in a similar experiment was shown that p53 could do so. This is another argument that apoptin seems not to act via the p53-apoptotic pathway.

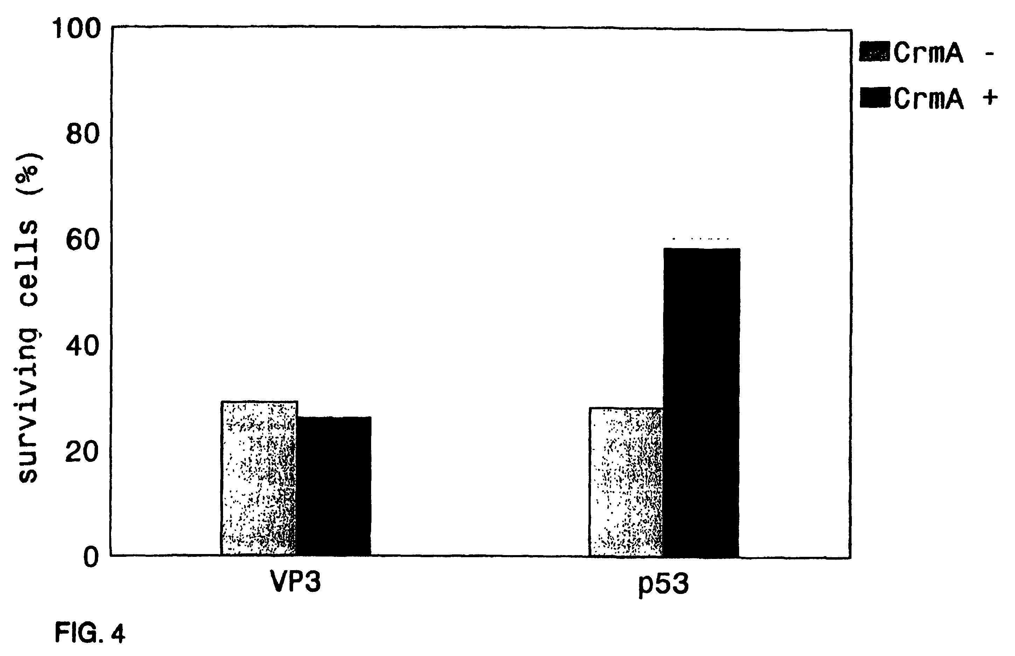

Interleuking-1-beta-converting-enzyme (ICE)-like proteins are known to be the last or one of the last steps in the decision cascade of the apoptotic process (Kumar, 1995). To examine whether ICE-like proteins might play a role in apoptin-induced apoptosis, the ICE-inhibitor crmA was co-expressed with apoptin. We have obtained evidence that inhibition of the ICE-like proteins due to expression of the cowpox protein crmA does not result in inhibition of apoptin-induced apoptosis. However, in a parallel experiment it was shown that crmA expression could reduce p53-induced apoptosis (Figure 4).

Therefore, we conclude that apoptin is very close to or even beyond the point of no-return within the apoptotic decision cascade. Furthermore, apoptin-induced apoptosis do not or only use a very minor part of the p53-induced apoptotic pathway.

### Expression of apoptin in normal human cells.

Three types of human primary cells, i.e. vascular endothelial cells (HUVEC), smooth muscle cells (HSMC) and T cells, were transiently transfected with a plasmidencoding full-sized apoptin (pCMV-fs). Cells expressing apoptin were screened via indirect immunofluorescence with MAb 85.1[8] or 111.3. Induction of apoptosis in apoptin-positive cells, was analysed with the help of DAPI or PI, which stain intact nuclei regularly, but apoptotic ones irregularly and/or weakly (Telford, 1992). Five days after transfection, only around 20% of the apoptin-expressing primary cells stained abnormally with DAPI or PI (data not shown). Menke et al. (unpublished) data have reported for another apoptosis system that this low percentage DAPI-abnormal cell is not due to a specific apoptosis-inducing agent, but due to transfection events. Also, the experiments shown below prove this statement. In similar experiments, 60-90% of malignant cells containing apoptin were apoptotic [Zhuang et al., 1995, 1995a,b). The localization of apoptin in these primary cells also differed from the localization in tumor cells. In all of the normal cells, apoptin was found in the cytoplasm, and not in the nucleus as has been observed for various tumor cell lines (Zhuang et al., 1995, 1995a,b). These results suggest that apoptin fails to induce apoptosis in various cultured human non-transformed, non-tumorigenic cells, and that the cellular location of apoptin is important for its apoptotic activity.

### Expression of apoptin in human normal cells and their malignant derivatives.

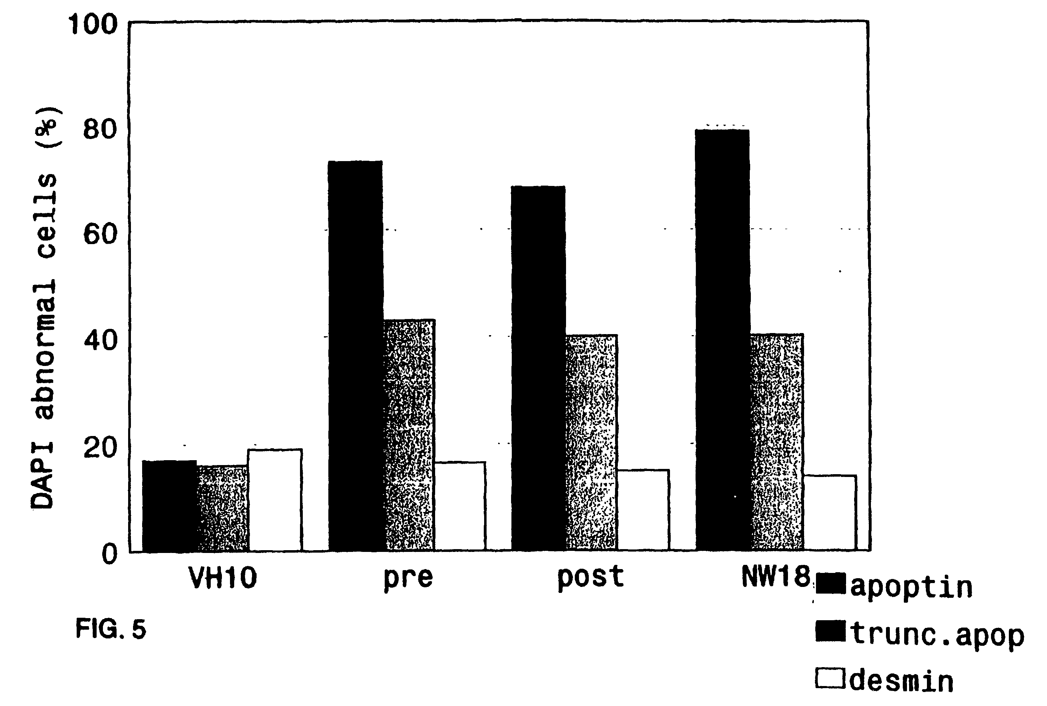

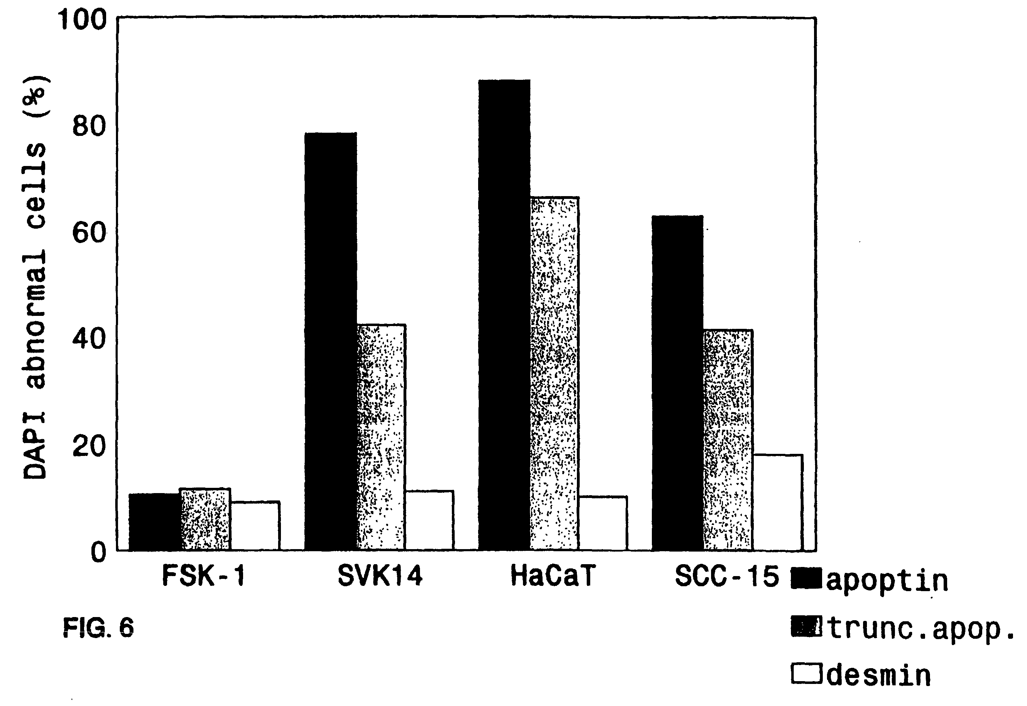

We also examined the response to apoptin expression in tumof cell lines versus that in the normal cells from which they had been generated. To that end, diploid skin fibroblasts and keratinocytes from normal individuals and their tumorigenic counterparts were transfected transiently with pCMV-fs, expressing full-sized apoptin, pCMV-tr, expressing truncated apoptin or a plasmid encoding desmin (pCMV-des). Desmin has no apoptotic activity (Menke et al. unpublished data), and is used as a negative control. Five days after transfection, the percentage of apoptin-positive VH10 fibroblasts (Figure 5) and FSK-1 keratinocytes (Figure 6), which had become apoptotic was not above 15%. This level of aberrantly DAPI-stained cells was similar for cells containing truncated apoptin or desmin. The low-level of apoptosis in these apoptin-positive cells may be due not to apoptin-specific induction of cell death, but to transfection events.

To examine whether tumorigenic fibroblasts and keratinocytes were susceptible to apoptin, NW18 and SCC-15 were transfected with plasmids encoding apoptin (full-sized or truncated) or desmin. Apoptin and, to a lesser extent, truncated apoptin could induce apoptosis in the NW-18 (Figure 5) and SCC-15 (Figure 6) tumor cells. Up to 65-75% of these apoptin-positive cells was apoptotic at five days after transfection, which is in the similar range as reported for apoptin-induced cell death in osteosarcoma cell lines (Zhuang et al., 1995a). The level of abnormal DAPI-stained desmin-positive NW-18 and SCC-15 cells was not significantly higher than that among normal fibroblasts and keratinocytes (Figures 5 and 6).

The differential activity of apoptin in normal and tumor cells cannot be explained by different proliferation rates, as these are similar for VH10 fibroblasts and Saos-2 osteosarcoma cells.

Our observations show that apoptin does not induce apoptosis in normal fibroblasts and keratinocytes, but does in their tumorigenic derivatives.

### Expression of apoptin in human transformed cells.

Is tumorigenicity required for apoptin to induce cell death, or is mere transformation of a cell sufficient? To answer this question we have examined the effect of apoptin in transformed,non-tumorigenic fibroblasts and keratinocytes. Apoptin, truncated apoptin, and desminwere transiently expressed in SV40-transformed fibroblasts, prior (Pre) or post (Post) immortalization, in SV40-transformed and immortalized SVK14 keratinocytes, and in spontaneously transformed HaCaT keratinocytes. Apoptin was able to induce apoptosis in all these types of transformed cell, to an extent similar to that in tumor cells (Figures 5 and 6). The percentage of cells containing (truncated) apoptin which had become apoptotic was significantly higher than in desmin-positive cells. These data imply that apoptin can induce apoptosis in both malignant and transformed cells.

Some chemotherapeutic agents and radiation can induce apoptosis in transformed cells but fail to do so in untransformed cells (Thompson, 1995, McDonell et al., 1995). Transformation seems to cause changes that make a cell more sensitive to apoptotic stimuli. Some other proteins are known to kill tumor cells specifically. The parvovirus structural protein NS-1 induces cell lysis specifically in neoplastic cells (VanAcker and Rommelaere, 1995). Also, a bcl-xs-expressing adenovirus has been constructed that induces apoptosis in human breast- and colon-carcinoma and neuroblastoma cells, but not in human normal hematopoietic cells or in the human leukemia cell line K562 (Clarke et al., 1995).

### Stable transfection of VH10 cell with a plasmid ecoding apoptin.

To exclude that apoptin has a minor but significant apoptotic activity in normal VH10 cells, these cells were stably transfected with pCMV-fs or with pCMV-neo-Bam as a control. We obtained a similar amount of colonies in both transfections. The resulting cells, stably transfected with pCMV-fs, expressed apoptin which was located in the perinuclear region (data not shown). Therefore, it can be concluded that apoptin cannot induce apoptosis or inhibit the growth of normal VH10 cells in any other sense.

### Cellular localization of apoptin in normal versus transformed and malignant cells.

Apart from the different apoptotic activities of apoptin in malignant and transformed cells versus normal cells, we have also observed differences in the cellular localization of apoptin in these cell types. In transformed and malignant cells, before the apoptotic morphological changes are oticeable, apoptin is, distributed as fine granules, mainly in the nucleus. After the cells had undergone apoptosis, apoptin was aggregated in the nucleus. In contrast, the location of apoptin in normal fibroblasts and keratinocytes is mainly in the cytoplasm, concentrated round the nucleus, both as small granules and larger aggregates. In primary HUVECs, HSMCs and T cells, apoptin was also localized in these characteristic perinuclear structures. Thus far, in all malignant or transformed cell lines studied, apoptin had a nuclear location, whereas in all normal cells analysed thusfar, it had not.

Others have proposed that transformed cells have undergone loss-of-function mutations, as a result of which a normally functioning inhibitor has been switched off. Due to these changes, nuclear transport of proteins may be promoted or hindered in cancer cells (Csermely et al., 1995). It is possible that in normal cells apoptin is associated to and/or modified by one or more cellular factor(s), resulting in its location within perinuclear structures. The loss of such (functional) factor(s) in malignant cells might allow apoptin to enter the nucleus. Apoptin harbors not only putative nuclear-import sequences (Noteborn et al., 1991, 1994, 1995, Zhuang et al., 1995, 1995a,b, but also an amino-acid region resembling nuclear export signals (position 33-46: IRIGIAGITITLSL), similar to that of the protein kinase inhibitor (PKI) and the HIV-Rev protein ( Wen et al., 1995, Fischer, et al., 1995, Gerace, 1995). It might well be that this potential nuclear export signal cannot be recognized in the various analysed malignant and transformed cell lines.

The results described here indicate that the nuclear localization of apoptin is important for its ability to induce apoptosis. This is in agreement with the observation that truncated apoptin, which has a reduced apoptotic activity (Figures 5 and 6), is partially in the cytoplasm. Electron microscopic studies with chicken mononuclear cells revealed that apoptin co-localizes with the cellular chromatin (Noteborn et al., 1994). The interaction of apoptin with chromatin could result in unwinding of its superstructure. The latter phenomenon has been described for rat ventral prostate cells, which became apoptotic after castration of the rats (Kyprianou and Isaacs, 1989).

### Apoptin as an anti-tumor agent.

Our results indicate that apoptin is an anti-tumor agent. Firstly, apoptin is specifically active in malignant and transformed cells, but, at least in vitro, not in the normal cells tested. Therefore, the toxic effect of apoptin treatment might be very low. Secondly, apoptin induces apoptosis in a p53-independent manner. The fact that several chemotherapeutic agents lose this capacity to induce apoptosis in a large number of tumors seems to be related to a disruption of p53 function. Therefore, induction of a p53-independent apoptotic pathway might be a useful approach as an alternative candidate for tumor therapy. Furthermore, apoptin apparently is not blocked by Bcl-2, which is known to be involved in the development of, e.g., leukemic tumors and which can inhibit apoptosis induced by chemotherapeutic agents. In addition, we have observed that BAG-1, a Bcl-2-related protein, is not able to block apoptin-induced apoptosis.

### Description of the figures

Figure 1 shows the effect of expression of E1B-21kD and Bcl-2 on the induction of apoptosis by VP3 in p53-minus Hep3B cells. The percentage given is that of the apoptin-positive cells which are apoptotic 6 days after transfection. The cells were co-transfected with 2.5 ug of pCMV-VP3 and 5 ug of pCMV-neo-Bam (open bars), 5 ug of pCMV-E1B21 (hatched bars), or 5 ug of pCMV-Bcl2 plasmid DNA (dotted bars). At least 3 independent experiments were carried out. Per experiment at least 200 apoptin-positive cells were examined.
Figure 2 shows the effect of E1B-21K and Bcl-2 proteins on the induction of apoptosis by p53 in the Hep3B cell line. The cells were co-transfected with 2.5 Êug of pCMV-p53 and 5 Êug of pCMV-neo-Bam, 5 ug of pCMV-E1B21K, or 5 ug of pCMV-Bcl2 plasmid DNA. Two independent transfections were carried out. The cells were analysed 4 or 5 days after transfection. The percentage given is that of p53-positive cells which are apoptotic. Per experiment at least 200 p53-positive cells were examined.
Figure 3 shows the effect of Bcl-2 and Bag-1 expression on the p53-induced apoptosis or apoptin-induced apoptosis. Saos-2 cells were co-transfected with 2.5 ug of pCMV-fs, and 5 ug of pCMV-Bcl-2, pCMV-Bag-1, or PCMV-Bag-1 and pCMV-Bcl-2. In a parallel experiment, the cells were co-transfected with 2.5 ug of pCMV-p53 and 5 ug pCMV-Bcl-2, pCMV-Bag-1 or pCMV-Bag-1 and pCMV-Bcl-2. As controls, pCMV-p53 or pCMV-fs were co-transfected with pCMV-neo-Bam. At least, 3 independent experiments of both series were carried out. The cells were harvested 4 days after transfection. The percentage is given of the p53- or apoptin-positive cells which have become apoptotic. Per experiment at least 200 cells have been examined.
Figure 4 shows the effect of the ICE-inhibitor crmA on the induction of p53- or apoptin-induced apoptosis. Saos-2 cells were co-transfected with 2.5 ug pCMV-fs and 5 ug pCMV-crmA (+crmA) or pCMV-neo-Bam (-crmA). In parallel, cells were co-transfected with 2.5 ug pCMV-p53 and 5 ug pCMV-crmA or pCMV-neo-Bam. Two independent experiments were carried out. The cells were harvested 5 days after transfection. The percentage is given of the p53- or apoptin-positive cell which have become apoptotic. Per experiment, at least 200 positive cells were examined.
Figure 5 shows the apoptin activity in normal versus transformed or malignant human fibroblasts. The percentage of cells that stained abnormally with DAPI is given as a relative measure for apoptosis in normal VH10 versus transformed (Pre, Post) and tumor (NW18) fibroblasts, transiently transfected with pCMV-fs, pCMV-tr or pCMV-des. Cells were fixed five days after transfection and analysed by indirect immunofluorescence. Results are the means of at least three independent experiments. In each experiment, at least 200-full-sized or truncated apoptin-or desmin-positive cells were examined. Cells were fixed 5 days after transfection and analysed by indirect immunofluorescence.
Figure 6 shows the apoptin activity in normal versus transformed or malignant keratinocytes. The percentage of cells that stained abnormally with DAPI is given as a relative measure for apoptosis in normal keratinocytes (FSK-1) versus transformed (SVK14, HaCAT) and tumor (SCC-15) ones, transiently transfected with pCMV-fs, pCMV-tr, or pCMV-des. Cells were fixed five days after transfection and analysed by indirect immunofluorescence. Results are the means of at least three independent experiments. In each experiment, at least 200-full-sized or truncated apoptin- or desmin-positive cells were examined.

### REFERENCES

1. Arends, M.J. and Wyllie, A.H. 1991. Apoptosis: mechanisms and roles in pathology. International Review of Experimental Pathology 32, 223-254.
2. Baker, S.J., Markowitz, Fearon, E.R., Wilson, J.K.V., and Vogelstein, B. 1990. Suppression of human colorectal carcinoma cell growth by wild-type p53. Science 245, 912-915.
3. Bellamy, C.O.C., Malcomson, R.D.G., Harrison, D.J., yllie, A.H. 1995. Cell death and disease: the biology and regulation of apoptosis. Seminars on Cancer Biology 6, 3-12.
4. Boukamp, P., Petrussevska, R.T., Breitkreutz, Hornung, J., Markham, A. and Fusenig, N. 1988. Normal keratinization in a spontaneouly immortalized aneuploid human keratinocyte cell line. Journal of Cell Biology 106, 761-771.
5. Chiou, S.-K., Rao, L., and White, E. 1994. Bcl-2 blocks p53-dependent apoptosis. Molecular Cellular Biology 14, 2556-2563.
6. Clarcke, M.F., Apel, I.J., Benedict, M.A., Eipers, P.G., Sumantran, V. et al. 1995. A recombinant bcl-Xs adenovirus selectively induces apoptosis in cancer cells but not in normal bone marrow cells. Proceedings of National Academy of Sciences USA 92, 11024-11028.
7. Csermely, P., Schnaider,T. and Szanto, I. Signalling and transport through the nuclear menbrane. 1995. Biochimica Biophysica Acta 1241, 425-452.
8. Danos, O., and Mulligan, R.C. (1988). Safe and efficient generation of recombinant retroviruses with amphotropic and ecotropic host ranges. Proceedings of National Academy Sciences, USA 85, 6460-6464.
9. Debbas, M. and White, E. 1993. Wild-type p53 mediates apoptosis by E1A, which is inhibited by E1B. Genes and Development 7, 546-554.
10. Earnshaw, W.C., 1995. Nuclear changes in apoptosis. Current Opinion in Cell Biology 7, 337-343.
11. Fisher, D.E. 1994. Apoptosis in Cancer therapy: crossing the treshold. Cell 78, 539-542.
12. Fischer, U., Huber, J., Boelens, W.C., Mattaj, I.W., LÅhrmann, R. 1995. The HIV-1 rev activation domain is a nuclear export signal that accesses an export pathway used by specific cellular RNAs. Cell 82, 475-483.
13. Gerace, L. 1995. Nuclear export signals and the fast track to the cytoplasm. Cell 82, 341-344.
14. Graham, F.L. and Van der Eb, A.J. 1973. A new technique for the assay of infectivity of human adenovirus 5 DNA. Virology 52, 456-467.
15. Hockenberry, D.M. 1994. Bcl-2 in cancer, development and apoptosis. Journal of Cell Science, Supplement 18, 51-55.
16. Hooper, M.L. 1994. The role of the p53 and Rb-1 gene in cancer, development and apoptosis. Journal Cell Science, Supplement 18, 13-17.
17. Kaufman, S.H. 1989. Induction of endonucleolytic DNA cleavage in human acute myelogenous leukemia cells by etoposide, camptothecin, and other cytotoxic anticancer drugs: a cautionary note. Cancer Research 49, 5870-5878.
18. Kerr, J.F.R., Winterford, C.M. Harmon, B.V. 1994. Apoptosis: Its significance in cancer and cancer therapy. Cancer 73, 2013-2026.
19. Klein, B., Pastink, A., Odijk, H., Westerveld, A. and Van der Eb, A.J. 1990. Experimental Cell Research 191, 256-262.
20. Kumar, S. 1995. ICE-like proteases in apoptosis. Trends in Biochemical Sciences 20, 198-202.
21. Kyprianou, N. and Isaacs, J.T. 1989. Activation of programmed cell death in the rat ventral prostate after castration. Endocrinology 122, 522-532.
22. Lowe, S.W., Ruley, H.E., Jacks, T., and Housman, D.E. 1993. p53-dependent apoptosis modulates the cytotoxicity of anticancer agents. Cell 74, 957-967.
23. Lowe S.H., Bodis, McClatchey, Remington, L., Ruley, H.E., Fisher, D.E., Housman, Jacks, T. 1994. p53 status and the efficacy of cancer therapy in vivo. Science 266, 807-810.
24. Maniatis, T., Fritsch, E.F., and Sanbrook, J. 1982. Molecular Cloning: A Laboratory Manual. CSHL Press, New York, USA.
25. McDonell, T.J., Meyn, R.E., Robertson, L.E. 1995. Implications of apoptotic cell death regulation in cancer therapy. Seminars in Cancer Biology 6, 53-60.
26. Noteborn, M.H.M. and De Boer, G.F. 1991. Patent application USA/no. 030, 335.
27. Noteborn, M.H.M. and Koch, G. 1994. Patent application no. PCT/NL94/00168.
28. Noteborn, M.H.M., De Boer, G.F., Van Roozelaar, D.J., Karreman, C., Kranenburg, O., Vos, J.G., Jeurissen, S.H.M., Hoeben, R., Zantema, A., Koch, G., Van Ormondt, H., and Van der Eb. 1991. Characterization of cloned chicken anemia virus DNA that contains all elements for the infectious replication cycle. Journal of Virology 65, 3131-3139.
29. Noteborn, M.H.M. and Koch, G. 1995. Chicken anaemia virus infection: molecular bassis of pathogenicity. Avian Pathology. 24, 11-31.
30. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J. and Koch, G. 1994. Journal of Virology 68, 346-351.
31. Oltvai, Z.N., Millman, C.L. and Kormeyer, S.J. 1993. Bcl-2 heterodimerizes in vivo with a conserved homolog Bax, that accelerates programmed cell death. Cell 74, 609-619.
32. Rheinwald, J. and Beckett, M.A. 1980. Defective terminal differentiation in cultures as a consistent and selectable character of malignant human keratinocytes. Cell 22, 629-632.
33. Rheinwald, J.G. and Green, H. (1975). Serial cultivation of strains of human epidermal keratinocytes: the formation of keratinizing colonies from single cells. Cell 6, 331-343.
34. Sachs, L. and Lotem J. 1993. Control of programmed cell death in normal and leukemia cells: new implications for therapy. Blood 82, 15-21.
35. Smith, M.L. and A.J. Fornace Jr, 1995. Genomic instability and the role of p53 mutations in cancer cells. Current Opinion in Oncology 7, 69-75.
36. Steller, H. 1995. Mechanisms and Genes of cellular suicide. Science 267, 1445-1449.
37. Takayama, S., Sato, T., Krajeswki, S., Kochel, K., Irie, S., Millan, J.A., and Reed, J. 1995. Cloning and functional analysis of Bag-1: A novel Bcl-2-binding protein with anti-cell death activity. Cell 80, 279-284.
38. Taylor-Papadimitriou, J., Purkis, P., Lane, B., McKay, I. and Chang, S.E. 1982. Effects of SV40 transformation on the cytoskleton and behavioural properties of human keratinocytes. Cell Differentiation 11, 169-180.
39. Telford, W.G., King, L.E., and Fraker, P.J. 1992. Comparative evaluation of several DNA binding dyes in the detection of apoptosis-associated chromatin degradation by flow cytometry. Cytometry 13, 137-143.
40. Thompson, C.B. 1995. Apoptosis in the pathogenesis and treatment of disease. Science 267, 1456-1462.
41. Vanacker, J.M. and J. Rommelaere. 1995. Non-structural proteins of autonomous parvoviruses: from cellular effects to molecular mechanisms. Seminars in Virology 6, 1-6.
42. Weissman, B.E. and Stanbridge, E.J. 1983. Complexity of control of tumorigenic expression in intraspecies hybrids of human SV40-transformed fibroblasts and normal human fibroblast cell lines. Cytogenetics and Cell Genetics 35, 263-268.
43. Wen, W., Meinkoth, J.L., Tsien, R.Y., Taylor, S.S. 1995. Identification of a signal for rapid export of proteins from the nucleus. Cell 82, 463-473.
44. White, E. 1996. Life, death, and the pursuit of apoptosis. Genes and Development 10, 1-15.
45. Wyllie, A.H. 1995. The genetic regulation of apoptosis. Current Opinion in Genetics and Development 5, 97-104.
46. Wyllie, A.H., Kerr, J.F.R., Currie, A.R. 1980. Cell death: the significance of apoptosis. International Review of Cytology 68, 251-306.
47. Zhuang, S.-M., Landegent, J.E., Verschueren, C.A.J., Falkenburg, J.H.F., Van Ormondt, H., Van der Eb, A.J., and Noteborn, M.H.M. 1995. Apoptin, a protein encoded by chicken anemia virus, induces cell death in various hematologic malignant cell in vivo. Leukemia 9, S118-120.
48. Zhuang, S.-M., Shvarts, A., Van Ormondt, H., Jochemsen, A.G., Van der Eb, A.J., and Noteborn, M.H.M. 1995a. Apoptin, a protein derived from chicken anemia virus, induces p53-independent apoptosis in human osteosarcoma cells. Cancer Research 55, 486-489.
49. Zhuang, S.-M., Shvarts, A., Jochemsen, A.G., Van der Eb, A.J., and Noteborn. 1995b. Differential sensitivity for Ad5 E1B and Bcl-2 proteins of apoptin-induced cell death. Carcinogenesis 16, 2939-2944.

## Claims

1. A method for distinguishing between transformed and/or malignant and/or tumor human cells and normal human cells comprising the steps of providing said cells in vitro with viral protein 3 (VP3; apoptin) and detecting the presence or absence of apoptotic activities of apoptin in malignant and transformed human cells versus normal human cells.

2. Use of a vehicle for the preparation of a medicament for the prevention of malignancies, wherein said vehicle is used for delivering a nucleic acid of interest to a human transformed, non-immortalized target cell and wherein said vehicle comprises a gene encoding apoptin or a fragment or a derivative thereof which causes apoptosis in transformed human cells and not in normal human cells.

3. Use of a vehicle comprising a gene encoding a tumoricidal substance and a targeting moiety having binding affinity for a molecule associated mainly, but not exclusively with the surface of a human tumor cell, for the preparation of a medicament for targeted tumor therapy by delivering a gene encoding a tumoricidal substance to a molecule associated mainly, but not exclusively with a human tumor cell, **characterized in that** the tumoricidal substance is apoptin or a fragment or a derivative thereof which causes apoptosis in transformed human cells and not in normal human cells.

## Patentansprüche

1. Verfahren zum Unterscheiden zwischen transformierten und/oder malignen und/oder Tumorzellen des Menschen und normalen menschlichen Zellen, umfassend die Schritte des Bereitstellens der Zellen in vitro mit viralem Protein 3 (VP3; Apoptin) und des Nachweisens des Vorhandenseins oder Nichtvorhandenseins apoptotischer Aktivitäten von Apoptin in malignen und transformierten menschlichen Zellen im Vergleich zu normalen menschlichen Zellen.

2. Verwendung eines Vehikels für die Zubereitung eines Medikamentes zur Prävention von Malignitäten, wobei das Vehikel zum Transportieren einer Nukleinsäure von Interesse zu einer menschlichen, transformierten, nicht-immortalisierten Zielzelle verwendet wird und wobei das Vehikel ein für Apoptin kodierendes Gen oder ein Fragment oder ein Derivat davon umfasst, welches in transformierten menschlichen Zellen und nicht in normalen, menschlichen Zellen Apoptose verursacht.

3. Verwendung eines Vehikels umfassend ein Gen, das für eine tumorizide Substanz kodiert, und eine Zielhälfte mit Bindungsaffinität zu einem Molekül, das hauptsächlich, jedoch nicht ausschließlich, mit der Oberfläche einer menschlichen Tumorzelle in Zusammenhang steht, zur Zubereitung eines Medikamentes zur gezielten Tumortherapie durch Transportieren eines Gens, das für eine tumorizide Substanz kodiert, zu einem Molekül, das hauptsächlich, jedoch nicht ausschließlich, mit einer menschlichen Tumorzelle in Zusammenhang steht, **dadurch gekennzeichnet, dass** die tumorizide Substanz Apoptin oder ein Fragment oder ein Derivat davon ist, welches in transformierten, menschlichen Zellen und nicht in normalen menschlichen Zellen Apoptose verursacht.

## Revendications

1. Un procédé pour distinguer des cellules humaines tumorales et/ou malignes et/ou transformées des cellules humaines normales comprenant les étapes de fournir lesdites cellules in-vitro ayant la protéine virale 3 (VP3; apoptine) et détecter la présence ou l'absence des activités apoptotiques de l'apoptine dans les cellules humaines transformées et malignes par rapport aux cellules humaines normales.

2. Utilisation d'un transporteur pour la préparation d'un médicament pour la prévention des malignités dans laquelle ledit véhicule est utilisé pour fournir un acide nucléïque d'intérêt à une cible humaine transformée non-immortalisée et dans laquelle ledit véhicule comprend un gène codant pour l'apoptine ou un fragment ou un dérivé de celui-ci qui provoque l'apoptose dans les cellules humaines transformées et non pas dans les cellules humaines normales.

3. Utilisation d'un véhicule comprenant un gène codant pour une substance tumoricide et une entité ciblante ayant une affinité de liaison pour une molécule qui s'associe principalement mais pas exclusivement à la surface d'une cellule humaine tumorale, pour la préparation d'un médicament destiné à la thérapie par ciblage des tumeurs telles que la fourniture d'un gène codant pour une substance tumoricide à une molécule associée principalement mais pas exclusivement à une cellule humaine tumorale **caractérisé en ce que** la substance tumoricide et l'apoptine ou un fragment ou un dérivé de celle-ci qui provoque l'apoptose dans les cellules humaines transformées et non pas dans les cellules normales.
